(19) Europäisches Patentamt
European Patent Office
Office européen des brevets

(11) EP 2 479 554 A2

(12) **EUROPÄISCHE PATENTANMELDUNG**

(43) Veröffentlichungstag:
**25.07.2012 Patentblatt 2012/30**

(51) Int Cl.:
***G01N 19/02*** *(2006.01)*

(21) Anmeldenummer: **11195944.1**

(22) Anmeldetag: **28.12.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **21.01.2011 DE 102011000276**

(71) Anmelder: **Krones AG**
**93073 Neutraubling (DE)**

(72) Erfinder:
- **Wickenhöfer, Florian**
  **93444 Bad Kötzting (DE)**
- **Hross, Stefan**
  **93092 Barbing (DE)**
- **Forsthövel, Jochen**
  **93059 Regensburg (DE)**
- **Lappe, Ulrich**
  **93059 Regensburg (DE)**

(74) Vertreter: **Reichert, Werner Franz**
**Bismarckplatz 8**
**93047 Regensburg (DE)**

(54) **Vorrichtung und Verfahren zum bestimmen der Reibung zwischen Kunststoffhohlkörpern aus gleicher Materialzusammensetzung**

(57) Die Erfindung betrifft eine Vorrichtung (1) und ein Verfahren zum Bestimmen der Reibung zwischen Kunststoffhohlkörpern (2) aus gleicher Materialzusammensetzung. Hierzu ist mindestens ein Kunststoffhohlkörper (2S) parallel zu seiner Längsachse (LS) mittels beidseitiger Klemmbacken (4) starr eingespannt. Ein linear beweglicher Kunststoffhohlkörper (2B) liegt auf dem starr eingespannten Kunststoffhohlkörper (2S) auf. Der linear und nicht parallel zur Längsachse (LS) bewegliche Kunststoffhohlkörper (2B) wird mit einer Kraft ($F_G$) beaufschlagt. Der bewegliche Kunststoffhohlkörper (2B) drückt mit der Kraft ($F_G$) auf den starr eingespannten Kunststoffhohlkörper (2S). Der Halsbereich (3) des beweglichen Kunststoffhohlkörpers (2B) ist mit einer Kraftmesseinheit (6) verbunden.

1
F_G
13
9
8
SW
12
7
2
2B
6
15
3
F_Z
11
V-V
14
H
2
4
4
10
H
B
A-A
2S
A-A

Fig. 4

EP 2 479 554 A2

## Beschreibung

Die vorliegende Erfindung betrifft eine Vorrichtung zum Bestimmen der Reibung zwischen Kunststoffhohlkörpern aus gleicher Materialzusammensetzung.

Ferner betrifft die Erfindung ein Verfahren zur Bestimmung der Reibung zwischen Kunststoffhohlkörpern aus gleicher Materialzusammensetzung.

Aus dem Stand der Technik sind Vorrichtungen bekannt, mit denen ein Reibwert zwischen einem Körper und einem Untergrund aus einem anderen Material bestimmt werden kann. Ebenso sind aus dem Stand der Technik Vorrichtungen bekannt, mit denen es möglich ist, den Reibwert (Rollreibung) zwischen Kunststoffhohlkörpern gleicher Materialzusammensetzung zu bestimmen.

Die internationale Patentanmeldung WO 2007/094704 A1 offenbart ein System zur automatischen Kontrolle der Reibwerte von Vorformlingen, die bei einer Blasmaschine zur Herstellung von Kunststoffflaschen verwendet werden. Aus den Vorformlingen werden PET-Kunststoffflaschen hergestellt. Mit dem erfindungsgemäßen System ist es möglich, die Reibwerte zwischen den Vorformlingen automatisch zu kontrollieren und anhand der ermittelten Messwerte entsprechend Flüssigkeit zuzugeben, damit der Reibwert zwischen den Vorformlingen auf ein bestimmtes Maß gesetzt wird. Hierzu ist in einer Kammer ein rotierender Träger vorgesehen. In der Kammer wird ein bestimmter Bremswert gemessen, der an eine Kontrollstation weitergegeben wird, von der aus dann entsprechend Flüssigkeit in die Kammer eingebracht wird.

Die britische Patentanmeldung GB 2 187 560 A offenbart eine Vorrichtung zur Messung der Oberflächenreibung. Mit der Vorrichtung ist es möglich, die Reibkräfte, welche an der Oberfläche eines Behälters auftreten, zu bestimmen. Hierzu wird der Behälter gegenüber einem mit einer Kraft beaufschlagten Schub gedreht. Gemessen wird die Bremskraft, welche der Schub über einen Hebel auf einen Kraftwandler ausübt. Mit dem Verfahren ist es möglich, die unterschiedlichen Reibwerte am Umfang einer Dose in Abhängigkeit von den verschiedenen farblichen Beschichtungen der Dose zu bestimmen.

Die U.S.-Patentanmeldung US 2002/0194895 A1 offenbart ein Analysesystem, mit dem die Reibung zwischen zwei Flaschen ermittelt werden kann. Mit der hier vorgeschlagenen Vorrichtung ist es möglich, den Reibungskoeffizienten für Plastikflaschen zu bestimmen, die eine nicht planare Oberfläche aufweisen. Mit der Vorrichtung ist es möglich, den Reibungskoeffizienten von Kunststoffflaschen, bzw. Vorformlingen untereinander zu bestimmen. Hierzu tritt eine stationäre Probe in Kontakt mit einer sich drehenden Probe. Auf die stationäre Probe wird eine Kraft in Richtung der sich gerade drehenden Probe ausgeübt. Das notwendige Drehmoment wird mittels eines Computers im Zeitpunkt des Abgleitens der beiden Proben untereinander bestimmt. Ebenso wird die Größe des Drehmoments bestimmt, welche notwendig ist, um eine konstante Geschwindigkeit aufrecht zu erhalten. Von den Drehmomentmessungen errechnet der Computer den Reibungskoeffizienten zwischen zwei Materialproben.

Die Patentschrift DD 234 933 A1 betrifft ein Verfahren und eine Anordnung zur Ermittlung von Haft- und Gleitreibwerten. Die Prüfwerkstoffe sind Kraftfahrzeugreifen und die auf die Kraftfahrzeugreifen wirkende Fahrbahnoberfläche. Die Prüfwerkstoffe werden mittels gelenkiger Vorrichtungen auf eine für die Messung vorgesehene Reibfläche gepresst und bei konstantem Anpressdruck durch mechanisch wirkende Einrichtungen mittels der Schwerkraft verschoben. Beim Verschieben der Prüfwerkstoffe auf der Reibfläche werden die vertikale Druckkraft, die auf die Prüfwerkstoffe wirkt, und die zur Verschiebung der Prüfwerkstoffe erforderliche Zugkraft zur Ermittlung der Reibwerte gemessen.

Aufgabe der Erfindung ist, eine transportable Vorrichtung zu schaffen, mit der es möglich ist, an jedem beliebigen Ort die Haftreibung zwischen zwei Kunststoffhohlkörpern aus gleicher Materialzusammensetzung zu bestimmen. Die Haftreibung wird im allgemeinen Sprachgebrauch auch als Klebrigkeit bezeichnet.

Die obige Aufgabe wird durch eine Vorrichtung gelöst, die die Merkmale des Patentanspruchs 1 umfasst.

Ferner ist es Aufgabe der Erfindung, ein Verfahren bereitzustellen, mit dem die Haftreibung zwischen zwei Kunststoffhohlkörpern aus gleicher Materialzusammensetzung bestimmt werden kann. Das Verfahren soll auch vor Ort bei einem Kunden, wie z. B. bei einer Streck-Blasmaschine oder bei der Eingangskontrolle der Vorformlinge, verwendet werden können.

Die obige Aufgabe wird durch ein Verfahren gelöst, das die Merkmale des Patentanspruchs 11 umfasst.

Der Hintergrund der gegenwärtigen Erfindung ist, dass ab einem bestimmten Grad der Klebrigkeit von fertig geblasenen Kunststoffflaschen oder Vorformlingen eine Stausituation an diversen Engstellen oder Einlaufstellen einer Linie zur Herstellung von Kunststoffflaschen mittels des Streck-Blasverfahrens entstehen kann. Eine besonders anfällige Stelle für die Bildung einer Stausituation ist die Zuführung der Vorformlinge oder der Einlauf in eine Etikettiermaschine. Durch diese Stausituation kann es zu Ausfällen von Linien kommen. Im schlimmsten Fall können bestimmte Kombinationen aus Vorformlingen und Kunststoffflaschen erst gar nicht mittels einer Linie verarbeitet werden. Um die Verarbeitung jedoch zu gewährleisten, kann dies zu teueren Nachrüstarbeiten und zu Unzufriedenheit auf Seiten des Kunden führen.

Die erfindungsgemäßen Vorrichtungen zum Bestimmen der Reibung zwischen Kunststoffhohlkörpern aus gleicher Materialzusammensetzung sind so konzipiert, dass die Reibwerte zwischen Kunststoffflaschen und zwischen Vorformlingen gemessen werden können. Dabei können sich die Vorformlinge im Druckmesser zwischen 12mm und 40mm bewegen. Der Durchmesser der aus den Vorformlingen hergestellten Kunststoffflaschen kann sich in einem Bereich von 50mm bis 120mm bewegen, so dass der Reibwert zwischen diesen Kunststoffflaschen noch mit der erfindungsgemäßen Vorrichtung gemessen werden kann. Hinsichtlich der Länge der Vorformlinge, bzw. der Kunststoffflaschen gibt es für die Messung keinerlei Einschränkung.

Die erfindungsgemäße Vorrichtung ist so konzipiert, dass mindestens ein Kunststoffhohlkörper parallel zu seiner Längsachse mittels beidseitiger Klemmbakken starr in der Vorrichtung eingespannt ist. Auch den mindestens einen starr eingespannten Kunststoffhohlkörper liegt ein weiterer Kunststoffhohlkörper auf, der nicht parallel zur Längsachse des starr eingespannten Kunststoffhohlkörpers angeordnet ist. Dieser Kunststoffhohlkörper ist somit nicht parallel zur Längsachse des starr eingespannten Kunststoffhohlkörpers beweglich. Der bewegliche Kunststoffhohlkörper kann mit einer vordefinierten Kraft beaufschlagt werden, so dass er mit dieser Kraft auf den starr eingespannten Kunststoffhohlkörper aufliegt. Mit einem Halsbereich des beweglichen Kunststoffhohlkörpers ist dieser mit einer Kraftmesseinheit verbunden.

Für den Fall, dass die Kunststoffhohlkörper Vorformlinge sind, sind zwei Vorformlinge zwischen den beidseitigen Klemmbacken der Vorrichtung starr eingespannt. Ein weiterer Vorformling ist dabei senkrecht zur Längsachse der starr eingespannten Vorformlinge beweglich angeordnet und liegt auf den beiden starr eingespannten Vorformlingen auf.

Für den Fall, dass die Kunststoffhohlkörper aus Vorformlingen hergestellte Kunststoffflaschen sind, reicht es aus, dass eine Kunststoffflasche zwischen den beidseitigen Klemmbacken der Vorrichtung starr eingespannt ist. Eine weitere Kunststoffflasche liegt senkrecht zur Längsachse der starr eingespannten Kunststoffflasche beweglich auf der starr eingespannten Kunststoffflasche auf. Für die Messung sind beide Kunststoffflaschen mit einem Innendruck beaufschlagt. Die mit dem Innendruck beaufschlagten Kunststoffflaschen sind mit einem entsprechenden Verschluss verschlossen, damit der Innendruck in den Kunststoffflaschen zumindest für die Zeit der Messung konstant bleibt.

Der mindestens eine starr eingespannte Kunststoffhohlkörper und der senkrecht zur Längsachse des starr eingespannten Kunststoffhohlkörpers bewegliche Kunststoffhohlkörper werden mit einer Kraft $F_G$ (Gewichtskraft) beaufschlagt, und so gegeneinander gedrückt. Um die Kraft aufzuwenden, ist ein schwenkbarer Hebelarm vorgesehen, der an einem freien Ende mindestens ein frei drehbares Lager trägt. Über eine Schwenkachse ist der Hebelarm mit einem Gestell der Vorrichtung schwenkbar verbunden. Das mindestens eine frei drehbare Lager liegt dabei am beweglichen Kunststoffhohlkörper an. Zur Ausübung einer vordefinierten Kraft, mit der die beiden Kunststoffhohlkörper in der Vorrichtung gegeneinander gedrückt werden, kann der schwenkbare Hebelarm mit Gewichten versehen werden, um somit die erforderliche vordefinierte Kraft auszuüben.

Gemäß einer weiteren Ausführungsform kann der schwenkbare Hebelarm über eine Schwenkachse mit dem Gestell der Vorrichtung schwenkbar verbunden sein. Ebenso kann der Hebelarm über ein Langloch in der Höhe in Bezug auf eine Basis der Vorrichtung linear verstellbar verbunden sein. Das freie drehbare Lager liegt dabei ebenfalls am beweglichen Kunststoffhohlkörper an. Mittels der Verstellung des schwenkbaren Hebelarms in der Höhe ist es möglich, dass der schwenkbare Hebel auf den Durchmesser der zu untersuchenden Kunststoffhohlkörper angepasst wird. Vorzugsweise ist der Hebelarm parallel zur Basis der Vorrichtung angeordnet.

Die erfindungsgemäße Vorrichtung besitzt einen Schlitten, der mit der Vorrichtung verbindbar ausgestaltet sein kann. Der Schlitten selbst ist in vertikaler Richtung verstellbar ausgebildet. Dadurch ist es möglich, dass die Kraftmesseinheit in linearer Richtung des Schlittens verschiebbar ist, um somit eine Zugkraft auf den beweglichen Kunststoffhohlkörper auszuüben.

Die Kraftmesseinheit und der Halsbereich des beweglichen Kunststoffhohlkörpers sind mit einer starren Verbindung gekoppelt.

Zum besseren Transport der erfindungsgemäßen Vorrichtung ist diese modular ausgebildet. Die Vorrichtung besteht dabei aus einem ersten Modul und einem zweiten Modul, die lösbar miteinander verbunden werden können. Das erste Modul trägt dabei die Kraftmesseinheit. Das zweite Modul trägt die zu vermessenden Kunststoffhohlkörper, die Klemmbacken, mit denen mindestens ein Kunststoffhohlkörper starr eingespannt werden kann, und den schwenkbaren Hebelarm, mit dem der bewegliche Kunststoffhohlkörper mit einer vordefinierten Kraft beaufschlagt werden kann.

Mit dem erfindungsgemäßen Verfahren ist es möglich, die Reibung (Haftreibung) zwischen Kunststoffhohlkörpern aus gleicher Materialzusammensetzung zu bestimmen. Hierzu wird zunächst mindestens ein Kunststoffhohlkörper starr eingespannt. Ein weiterer Kunststoffhohlkörper, der letztendlich gegenüber dem starren eingespannten Kunststoffhohlkörper beweglich ist, wird auf den starr eingespannten Kunststoffhohlkörper gesetzt, so dass deren Längsachsen nicht parallel zueinander stehen. Der Halsbereich des beweglichen Kunststoffhohlkörpers wird mit einer Kraftmesseinheit verbunden. Der bewegliche Kunststoffhohlkörper wird mit einer Kraft beaufschlagt, so dass der bewegliche Kunststoffhohlkörper gegen den starr eingespannten Kunststoffhohlkörper gedrückt wird. Die Kraftmesseinheit wird schließlich linear verschoben und ein Maximum der Zugkraft ermittelt. Das Maximum der Zugkraft entspricht derjenigen Kraft, bei der der bewegliche Kunststoffhohlkörper an den mindestens einen starr eingespannten Kunststoffhohlkörper zu gleiten beginnt und somit die Haftreibung zwischen den Kunststoffhohlkörpern überwindet. Das Maximum der aufzuwendenden Zugkraft wird bei unterschiedlichen Kräften bestimmt, mit denen die Kunststoffhohlkörper gegeneinander gedrückt werden und setzen sich aus zwei Teilkräften zusammen. Die erste Teilkraft wird zur Überwindung der Haftreibung benötigt und die zweite Teilkraft dient zum Beibehalten des Gleitens der Kunststoffhohlkörper.

Die einzelnen ermittelten Maxima der Zugkraft werden als Funktion der Kraft aufgetragen, mit der der bewegliche Kunststoffhohlkörper beaufschlagt wird. Anschließend werden die Punkte mittels einer Geraden verbunden und der resultierende Schnittpunkt mit der Ordinate zeigt die Haftreibung.

Für den Fall, dass mit dem erfindungsgemäßen Verfahren Kunststoffflaschen gemessen werden sollen, werden diese mit einem Innendruck beaufschlagt und am Halsbereich mit einem Ventilverschluss verschlossen.

Zur Ermittlung der Haftreibung zwischen dem mindestens einen starr eingespannten Kunststoffhohlkörper und dem beweglichen Kunststoffhohlkörper sind diese derart zueinander angeordnet, dass sich die Oberflächen in einem zylindrischen Abschnitt der Kunststoffhohlkörper berühren.

Es ist von besonderem Vorteil, wenn die erfindungsgemäße Vorrichtung eine handliche Größe besitzt und zerlegt werden kann, so dass diese in einem herkömmlichen Reisekoffer zur Einsatzstelle transportiert werden kann. Für alle erforderlichen Verstellungen der Vorrichtung hinsichtlich des Einspannens mindestens eines Kunststoffhohlkörpers sollen möglichst Schnellverschlüsse verwendet werden, um somit auf den Einsatz von zusätzlichen Werkzeugen verzichten zu können. Die mit dem beweglichen Kunststoffhohlkörper verbundene Kraftmesseinheit ist als Zug-Druckkraftmesser ausgebildet. Die Kraftmesseinheit ist ebenfalls an einem höhenverstellbaren Schlitten angebracht, damit diese ebenfalls auf den Durchmesser der zu prüfenden Kunststoffhohlkörper angepasst werden kann. Die Kraftmesseinheit ist mittels des Schlittens horizontal frei beweglich in Bezug auf die übrige Messvorrichtung gelagert. Zur Ermittlung der Haltekraft zwischen den zu vermessenen Kunststoffhohlkörpern ist der Schlitten in fixer Abzugsrichtung zu bewegen, bis sich die Kunststoffhohlkörper voneinander lösen. Diese gemessene Haltekraft kann auf dem Display des Kraftmessers abgelesen werden.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.

Figur 1 zeigt schematisch die Anordnung der zu vermessenden Kunststoffhohlkörper, falls diese als Vorformlinge ausgebildet sind, wobei ebenfalls das wirkende Kräftegleichgewicht eingezeichnet ist.

Figur 2 zeigt schematisch die Anordnung von Kunststoffhohlkörpern zueinander, für den Fall, dass diese aus Kunststoffflaschen ausgebildet sind, wobei ebenfalls das für die Messung wirkende Kräftegleichgewicht eingezeichnet ist.

Figur 3 zeigt schematisch die Vorrichtung zur Vermessung der Haftreibung zwischen Kunststoffhohlkörpern, wobei diese als Vorformlinge ausgebildet sind.

Figur 4 zeigt schematisch die Vorrichtung zur Messung der Haftreibung zwischen Kunststoffhohlkörpern, wobei diese als Kunststoffflaschen ausgebildet sind.

Figur 5 zeigt eine perspektivische Ansicht der erfindungsgemäßen Vorrichtung zur Vermessung der Haftreibung zwischen Kunststoffhohlkörpern, wobei diese als Vorformlinge ausgebildet sind.

Figur 6 zeigt eine perspektivische Ansicht des zweiten Moduls der Vorrichtung, in der die zu vermessenden Vorformlinge für die Messung eingesetzt werden.

Figur 7 zeigt eine perspektivische Ansicht eines Stapels aus Gewichten, die auf dem im zweiten Modul vorgesehenen Hebelarm aufgesetzt werden können.

Figur 8 zeigt eine Frontansicht des Hebelarms der erfindungsgemäßen Vorrichtung, bei dem ein Stapel aus Gewichten auf den Hebelarm aufgesetzt ist.

Figur 9 zeigt eine perspektivische Ansicht des Hebelarms.

Figur 10 zeigt eine perspektivische Ansicht von unten des ersten Moduls, das mit dem zweiten Modul verbunden werden kann.

Figur 11 zeigt eine perspektivische Draufsicht auf das erste Modul, welches die Kraftmesseinheit trägt.

Figur 12 zeigt eine perspektivische Ansicht der erfindungsgemäßen Vorrichtung, die für die Vermessung der Haftreibung zwischen Kunststoffhohlkörpern ausgestaltet ist, wobei die Kunststoffhohlkörper aus Vorformlingen gefertigten Kunststoffflaschen bestehen.

Figur 13 zeigt eine Draufsicht auf die in Figur 12 dargestellte Vorrichtung.

Figur 14 zeigt die perspektivische Ansicht der in Figur 12 gezeigten Vorrichtung, bei der ebenfalls die zu vermessenden Kunststoffflaschen in das zweite Modul der Vorrichtung eingesetzt sind.

Figur 15 zeigt eine perspektivische Ansicht des ersten Moduls der erfindungsgemäßen Vorrichtung, bei der der Schlitten zur Aufnahme der Kraftmesseinheit höhenverstellbar ausgebildet ist.

Figur 16 zeigt eine perspektivische Ansicht der Kraftmesseinheit, welche auf den Schlitten aufgesetzt werden kann.

Figur 17 zeigt anhand eines Diagramms die unterschiedliche Klebrigkeit der Vorformlinge in Abhängigkeit von deren Behandlung bei der Herstellung.

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dar, wie die erfindungsgemäße Vorrichtung und das erfindungsgemäße Verfahren ausgestaltet sein können und stellen keine abschließende Begrenzung der Erfindung dar.

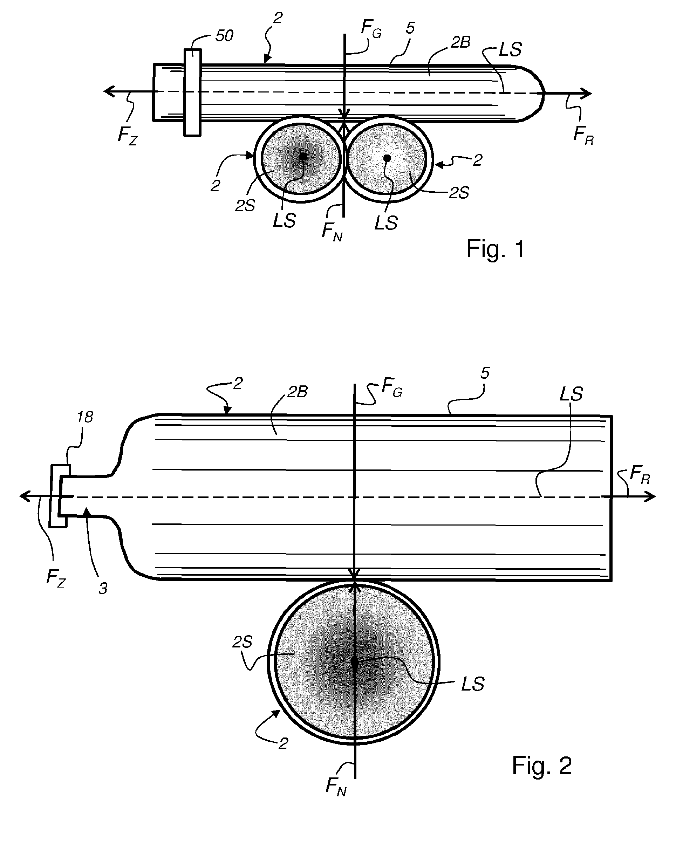

**Figur 1** zeigt schematisch die Anordnung der Kunststoffhohlkörper 2 zueinander, um die Haftreibung zwischen den einzelnen Kunststoffhohlkörpern 2 zu bestimmen. In der in Figur 1 gezeigten Ausführungsform sind die Kunststoffhohlkörper 2 als Vorformlinge ausgebildet. Die Kunststoffhohlkörper 2 sind dabei derart angeordnet, dass ein beweglicher Kunststoffhohlkörper 2B auf zwei starren Kunststoffhohlkörpern 2S aufliegt. Dabei ist die Längsachse LS des beweglichen Kunststoffhohlkörpers 2B senkrecht zu den Längsachsen der starren Kunststoffhohlkörper 2S ausgerichtet. Der bewegliche Kunststoffhohlkörper 2B ist somit mit einer im Wesentlichen zylindrischen Mantelfläche 5 in Kontakt mit der ebenfalls zylindrischen Mantelfläche 5 der starren Kunststoffhohlkörper 2S. Der bewegliche Kunststoffhohlkörper 2B wird mit einer Kraft $F_G$ beaufschlagt. Gegen diese Kraft $F_G$ wirkt eine Normalkraft $F_N$. An dem beweglichen Kunststoffhohlkörper 2B greift eine Zugkraft $F_Z$ an, der eine Haftreibungskraft $F_R$ entgegenwirkt.

**Figur 2** beschreibt die Anordnung der Kunststoffhohlkörper 2 für den Fall, dass diese als Kunststoffflaschen ausgebildet sind. Die Kunststoffflaschen werden aus Vorformlingen, wie in Figur 1 beschrieben, hergestellt. Bei der in Figur 2 gezeigten Anordnung der Kunststoffhohlkörper 2 für die Messung wirkt die gleiche Kräfteverteilung, wie bereits in Figur 1 beschrieben. Für den Fall, dass die Kunststoffhohlkörper 2 Kunststoffflaschen sind, werden diese für die Messung mit einem bestimmten Innendruck P gefüllt. Damit der Innendruck P zumindest für die Zeit der Messung konstant bleibt, ist der Kunststoffhohlkörper 2 an einem Halsbereich 3 mit einem Ventilverschluss 18 verschlossen. Die Zugkraft $F_Z$ greift somit am Halsbereich 3, bzw. am Ventilverschluss 18 des beweglichen Kunststoffhohlkörpers 2B an. Der bewegliche Kunststoffhohlkörper 2B und der starre Kunststoffhohlkörper 2S sind dabei derart zueinander angeordnet, dass deren Längsachsen LS senkrecht zueinander ausgerichtet sind und sich die Mantelflächen 5 des beweglichen Kunststoffhohlkörpers 2B und des starren Kunststoffhohlkörpers 2S berühren.

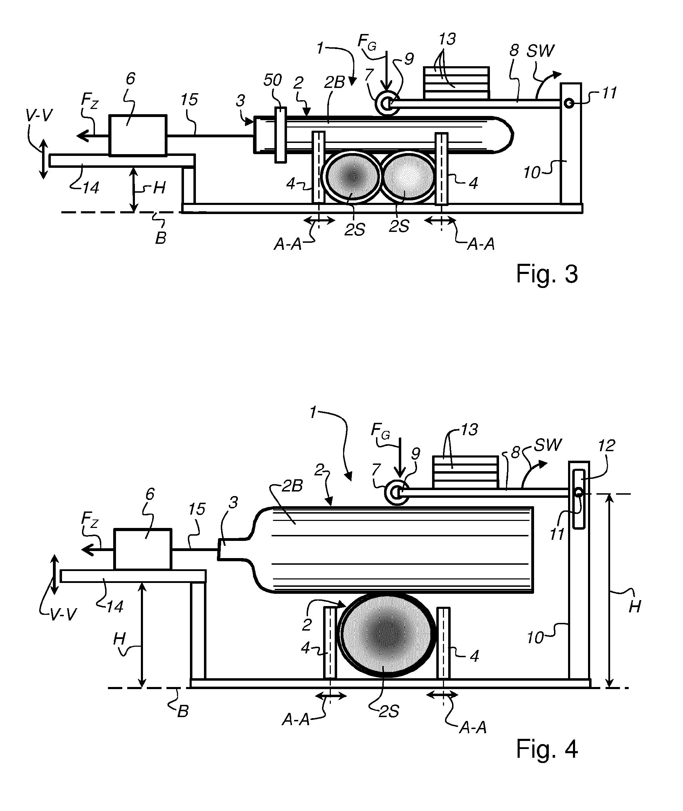

**Figur 3** zeigt einen schematischen Aufbau der erfindungsgemäßen Vorrichtung 1, mit der die Haftreibung zwischen zwei starr eingespannten Kunststoffhohlkörpern 2S und einem linear beweglichen Kunststoffhohlkörper 2B ermittelt wird. Für den Fall, dass die Kunststoffhohlkörper 2 als Vorformlinge ausgebildet sind, empfiehlt es sich, dass zwei Vorformlinge starr eingespannt werden. Für die Messung reicht es aus, dass ein Vorformling auf den starr eingespannten Vorformlingen aufliegt. Die Ausrichtung der Kunststoffhohlkörper 2 zueinander ist bereits in Figur 1 beschrieben. Die Kraft $F_G$, welche auf den beweglichen Kunststoffhohlkörper 2B ausgeübt wird, wird mittels eines schwenkbaren Hebelarms 8 erzeugt. Der schwenkbare Hebelarm 8 ist über eine Schwenkachse 11 mit dem Gestell 10 der Vorrichtung 1 verbunden. Am freien Ende 9 des schwenkbaren Hebelarms 8 ist mindestens ein Lager 7 angebracht. Das Lager 7 liegt auf dem beweglichen Kunststoffhohlkörper 2B auf. Das Lager 7, beispielsweise eine gelagerte Rolle, ist erforderlich, um eine freie Beweglichkeit des beweglichen Kunststoffhohlkörpers 2B gewährleisten zu können und somit keine zusätzlichen unerwünschten Kräfte auf die Anordnung der Kunststoffhohlkörper 2 für die Messung auszuüben. Die auf den beweglichen Kunststoffhohlkörper 2B wirkende Kraft $F_G$ kann dadurch variiert werden, dass unterschiedliche Gewichte 13 auf den Hebelarm 8 aufgesetzt werden. Die starren Kunststoffhohlkörper 2S können mittels mindestens zweier, in Richtung des Doppelpfeils A-A beweglicher Klemmbakken 4 in der Vorrichtung 1 eingespannt werden. Eine Kraftmesseinheit 6 ist auf einem Schlitten 14 montiert. Der Schlitten 14 kann entlang einer vertikalen Richtung V-V in der Höhe H in Bezug auf eine Basis B der Vorrichtung 1 verstellt werden. Die Verstellung des Schlittens 14 in der Höhe H ist erforderlich, damit die Zugkraft, welche mittels der Kraftmesseinheit 6 auf den beweglichen Kunststoffhohlkörper 2B ausgeübt wird, im Wesentlichen parallel zur Basis B der Vorrichtung 1 und zentral angreift. Der Halsbereich 3 des beweglichen Kunststoffhohlkörpers 2B ist mit der Kraftmesseinheit 6 mit einer starren Verbindung 15 verbunden. Damit die starre Verbindung 15 am Halsbereich 3 des beweglichen Kunststoffhohlkörpers 2B angreifen kann, kann der bewegliche Kunststoffhohlkörper 2B mit einer Verschlusskappe (hier nicht dargestellt) versehen sein. Zum Einsetzen neuer zu vermessender Kunststoffhohlkörper 2 kann der schwenkbare Hebelarm 8 mit der Schwenkachse 11 entlang der Schwenkrichtung SW geschwenkt werden.

**Figur 4** zeigt einen schematischen Aufbau der erfindungsgemäßen Vorrichtung 1, mit der Kunststoffhohlkörper 2 vermessen werden, die als Kunststoffflaschen ausgebildet sind, welche aus den Vorformlingen hergestellt werden. Der Aufbau der Vorrichtung 1 zur Vermessung der Haftreibung zwischen Kunststoffflaschen ist analog zum bereits in Figur 3 beschriebenen Aufbau. Ein Unterschied besteht darin, dass zusätzlich zum in der Höhe H verstellbaren Schlitten 14 ebenfalls der schwenkbare Hebelarm 8 über ein Langloch 12 in der Höhe H verstellt werden kann. Die Verstellung des Hebelarms 8 in Bezug auf die Höhe H zur Basis B der Vorrichtung 1 ist sinnvoll, damit der Hebelarm 8 ebenfalls parallel zur Basis B der Vorrichtung 1 auf den beweglichen Kunststoffhohlkörper 2B wirkt. Durch die Verstellbarkeit des schwenkbaren Hebelarms 8 in Bezug auf die Höhe H ist es möglich, sich auf die unterschiedlichen Durchmesser der zu vermessenden Kunststoffhohlkörper 2 einzustellen.

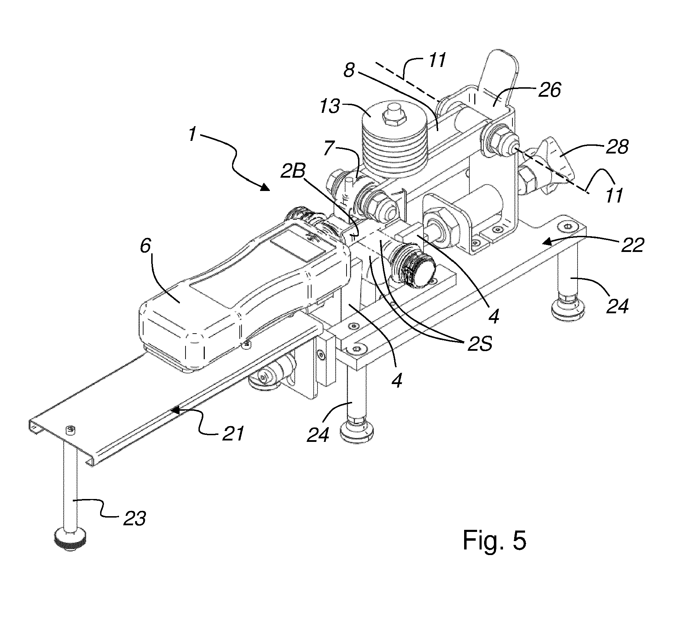

**Figur 5** zeigt eine perspektivische Ansicht einer Ausführungsform der erfindungsgemäßen Vorrichtung 1, die für die Vermessung von Kunststoffhohlkörpern 2 geeignet ist, falls diese Kunststoffhohlkörper 2 Vorformlinge sind. Die erfindungsgemäße Vorrichtung besteht aus einem ersten Modul 21 und einem zweiten Modul 22, die lösbar miteinander verbindbar sind. Das erste Modul 21 trägt die Kraftmesseinheit 6. Die Kraftmesseinheit 6 ist als Zug-Druckkraftmesser ausgebildet. Das zweite Modul 22 trägt die zu vermessenden Kunststoffhohlkörper 2, welche zwischen den mindestens zwei beweglichen Klemmbacken 4 starr eingespannt sind. Auf dem beweglichen Kunststoffhohlkörper 2B liegt der schwenkbare Hebelarm 8 mit dem Lager 7 auf. Auf dem schwenkbaren Hebelarm 8 können unterschiedliche Gewichte 13 aufgesetzt werden. Die Schwenkachse 11 des Hebelarms 8 ist in einem Formteil 26 gehaltert. Das Formteil 26 trägt ebenfalls eine Stellschraube 28, die auf die mindestens zwei beweglichen Klemmbacken 4 wirkt, um somit eine Klemmung der starren Kunststoffhohlkörper 2S zu erreichen. Das erste Modul 21 ist mit mindestens einem Fuß 23 versehen. Ebenso ist das zweite Modul 22 mit mindestens einem Fuß 24 versehen. Die Füße 23 und 24 sind ebenfalls in der Höhe verstellbar ausgebildet, so dass das erste Modul 21 und das zweite Modul 22 hinsichtlich der Höhe verstellbar und anpassbar sind.

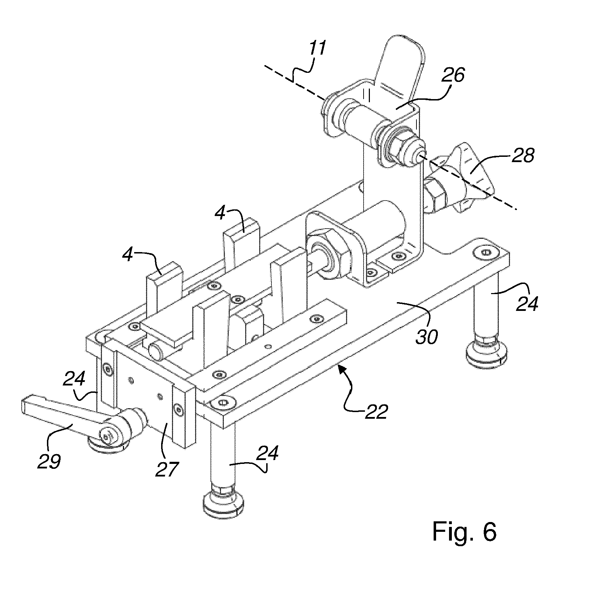

**Figur 6** zeigt eine perspektivische Ansicht des zweiten Moduls 22 der erfindungsgemäßen Vorrichtung 1 zur Vermessung der Haftreibung zwischen Vorformlingen, welche die zu vermessenden Kunststoffhohlkörper 2 darstellen. Wie bereits in Figur 5 beschrieben, ist auf einer Montageplatte 30 des zweiten Moduls 22 ein Formteil 26 angebracht. Das Formteil 26 trägt die Schwenkachse 11 für den Hebelarm (hier nicht dargestellt) und die Stellschraube 28 für die Bewegung der Klemmbakken 4, damit eine ausreichende Klemmung der starren Kunststoffhohlkörper 2S in der Vorrichtung 1 erzielt werden kann. Die Montageplatte 30 trägt ebenfalls die in der Höhe verstellbaren Füße 24. An der der Stellschraube 28 abgewandten Seite der Montageplatte 30 des zweiten Moduls 22 ist eine Anschlagplatte 27 angebracht, in der ein Schnellspannhebel 29 sitzt. Mit dem Schnellspannhebel 29 kann auf einfache und zeitsparende Weise das erste Modul 21 mit dem zweiten Modul 22 lösbar verbunden werden.

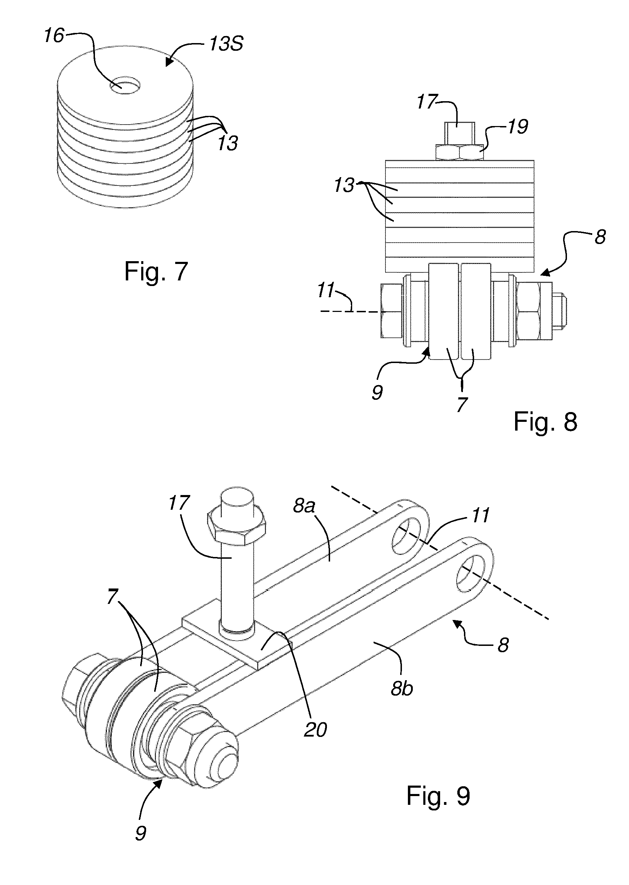

**Figur 7** zeigt eine perspektivische Ansicht eines Gewichtstapels 13S, welcher auf den schwenkbaren Hebelarm (hier nicht dargestellt) aufgesetzt werden kann, um die erforderliche Kraft $F_Z$ auf den beweglichen Kunststoffhohlkörper 2B auszuüben. Der Gewichtstapel 13S besteht aus einer Vielzahl von scheibenförmigen Einzelgewichten 13, bei denen jedes Gewicht 13 eine zentrale Bohrung 16 ausgeformt hat. Mittels der einzelnen Gewichte 13, welche unterschiedliches Gewicht oder gleiches Gewicht haben können, ist es möglich, unterschiedliche Kräfte $F_Z$ einzustellen, welche über den schwenkbaren Hebelarm 8 auf den beweglichen Kunststoffhohlkörper 2B wirken.

**Figur 8** zeigt eine Frontansicht einer Ausführungsform des Hebelarms 8, der bei der erfindungsgemäßen Vorrichtung 1 Verwendung findet. Am freien Ende 9 des Hebelarms 8, welches der Schwenkachse 11 abgewandt ist, trägt der Hebelarm 8 in der hier dargestellten Ausführungsform zwei freie drehbare Lager 7. Wie bereits erwähnt, liegen die frei drehbaren Lager 7 an der Mantelfläche 5 des beweglichen Kunststoffhohlkörpers 2B auf. Die einzelnen Gewichte 13 sind auf einen Dorn 17 aufgesetzt. Mit einer Mutter 19 werden die Gewichte 13 fest am Hebelarm 8 befestigt.

**Figur 9** zeigt eine perspektivische Ansicht des schwenkbaren Hebelarms 8, der bei der erfindungsgemäßen Vorrichtung 1 Verwendung findet. Der schwenkbare Hebelarm 8 besitzt einen ersten Arm 8a und einen zweiten Arm 8B. Der erste Arm 8a und der zweite Arm 8b sind über eine starre Brücke 20 miteinander verbunden. Die starre Brücke 20 trägt den Dorn 17 zur Aufnahme der verschiedenen Gewichte 13. Die beiden frei drehbaren Lager 7 sind ebenfalls zwischen dem ersten Arm 8a und dem zweiten Arm 8b des schwenkbaren Hebelarms 8 angeordnet.

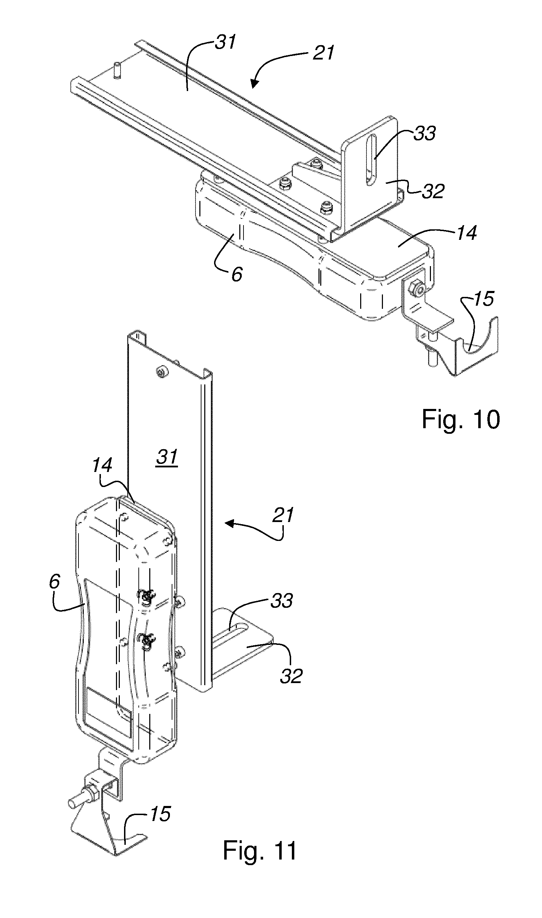

Die **Figuren 10** und **11** zeigen jeweils eine perspektivische Ansicht des ersten Moduls 21 aus verschiedenen Ansichten. Das erste Modul 21 umfasst eine Montageplatte 31, auf der der Schlitten 14 für die Kraftmesseinheit 6 sitzt. Auf dem Schlitten 14 ist die Kraftmesseinheit 6 in linearer Richtung beweglich. Zur Anbringung des ersten Moduls 21 an dem zweiten Modul 22 ist an der Montageplatte 31 des ersten Moduls 21 eine Platte 32 montiert. In der Platte 32 ist ein Langloch 33 ausgebildet. Durch dieses Langloch greift der Schnellspannhebel 29, mit dem das erste Modul 21 und das zweite Modul lösbar miteinander verbunden werden können. Mittels des Langlochs 33 ist es möglich, das erste Modul 21 in der Höhe in Bezug auf das zweite Modul 22 einzustellen. Die Kraftmesseinheit ist mit einer starren Verbindung 15 verbunden. Die starre Verbindung 15 dient dazu, dass die Verbindung von der Kraftmesseinheit 6 zum beweglichen Kunststoffhohlkörper 2B hergestellt wird. In der in Figur 10 und Figur 11 beschriebenen Ausführungsform ist die starre Verbindung 15 derart ausgebildet, dass sie z. B. hinter einem Tragring 50 (hier nicht dargestellt) eines Vorformlings greift.

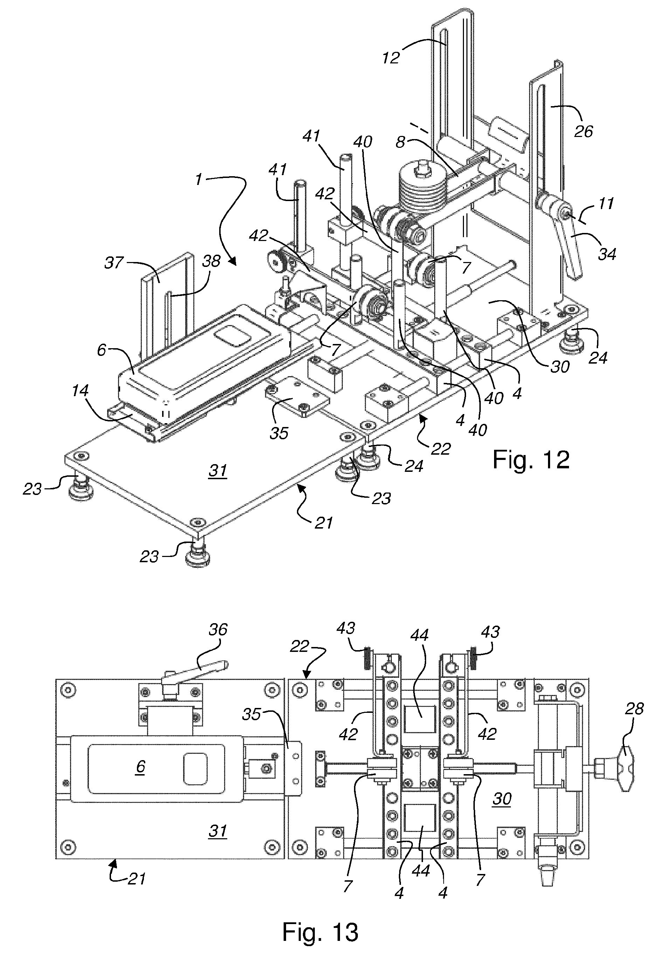

**Figur 12** zeigt eine perspektivische Ansicht einer Vorrichtung zur Bestimmung der Haftreibung zwischen Kunststoffhohlkörpern 2, wobei die Vorrichtung für Kunststoffhohlkörper 2 geeignet ist, die als Kunststoffflaschen ausgebildet sind. Die Kunststoffflaschen werden aus Vorformlingen hergestellt. Die erfindungsgemäße Vorrichtung 1 besteht ebenfalls aus einem ersten Modul 21 und einem zweiten Modul 22. Das erste Modul 21 besteht aus einer Montageplatte 31, die mit mehreren Füßen 23 versehen ist. An der Montageplatte 31 ist eine Halteplatte 37 angebracht, die ein Langloch 38 ausgebildet hat. An der Halteplatte 37 kann der Schlitten 14 für die Kraftmesseinheit 6 angebracht werden. Über das Langloch 38 ist der Schlitten 14 zusammen mit der Kraftmesseinheit 6 in der Höhe verstellbar ausgebildet. Mit einer Lasche 35 kann das erste Modul 21 mit dem zweiten Modul 22 der Vorrichtung 1 verbunden werden. Das zweite Modul 22 ist für die Vermessung der Haftreibung zwischen Kunststoffhohlkörpern 2 ausgebildet, die in Form von Kunststoffflaschen vorliegen. Auf der Basisplatte 30 des zweiten Moduls 22 sitzt ebenfalls ein Formteil, in dem ein Langloch 12 ausgebildet ist. In dem Langloch 12 kann die Schwenkachse 11 des Hebelarms 8 in der Höhe verstellt werden. Mittels eines Schnellspannhebels 34 kann die so eingestellte Höhe des schwenkbaren Hebelarms 8 fixiert werden. In die beweglichen Klemmbacken 4 können an verschiedenen Positionen Stifte eingesetzt werden, die letztendlich den starren Kunststoffhohlkörper 2S klemmen. An jeweils einem Ende der Klemmbacken 4 ist ein Stab 41 eingesetzt. An jedem Stab 41 ist ein höhenverstellbarer Tragarm 42 angebracht. Jeder der höhenverstellbaren Tragarme 42 trägt ebenfalls ein Lager 7. Die höhenverstellbaren Tragarme 42 können derart eingestellt werden, dass der bewegliche Kunststoffhohlkörper 2B auf den Lagern 7 der Tragarme 42 aufliegt und gleichzeitig mit der Mantelfläche 5 des starren Kunststoffhohlkörpers 2S in Kontakt ist.

**Figur 13** zeigt eine Draufsicht auf die erfindungsgemäße Vorrichtung 1, die zur Ermittlung der Haftreibung zwischen Kunststoffhohlkörpern 2 ausgebildet ist, die die Form von Kunststoffflaschen haben. Wie bereits in der Beschreibung zu Figur 12 erwähnt, liegt die Kraftmesseinheit 6 auf einem Schlitten 14. Der Schlitten 14 kann entlang einer Halteplatte in der Höhe H verstellt werden. Zur Fixierung der richtig eingestellten Höhe H ist ein Schnellspannhebel 36 vorgesehen. Das zweite Modul 22 trägt auf der Montageplatte 30 die verstellbaren Klemmbacken 4. Mittels der Stellschraube 28 können die Klemmbacken 4 aufeinander zu und voneinander weg bewegt werden. Die in den Klemmbacken 4 sitzenden Stifte 40 halten somit den zu untersuchenden Kunststoffhohlkörper 2, bzw. Kunststoffflasche. Die in Richtung der Kraftmesseinheit 6 bewegliche Kunststoffflasche liegt auf den Lagern 7 auf, die an entsprechenden Haltearmen 42 angebracht ist. Die Haltearme 42 können mittels der Stäbe 41 in der Höhe verstellt werden. Mittels Stellschrauben 43 können die Haltearme 42 in der vorgeschriebenen Höhe H für die gerade zu untersuchenden Kunststoffflaschen fixiert werden.

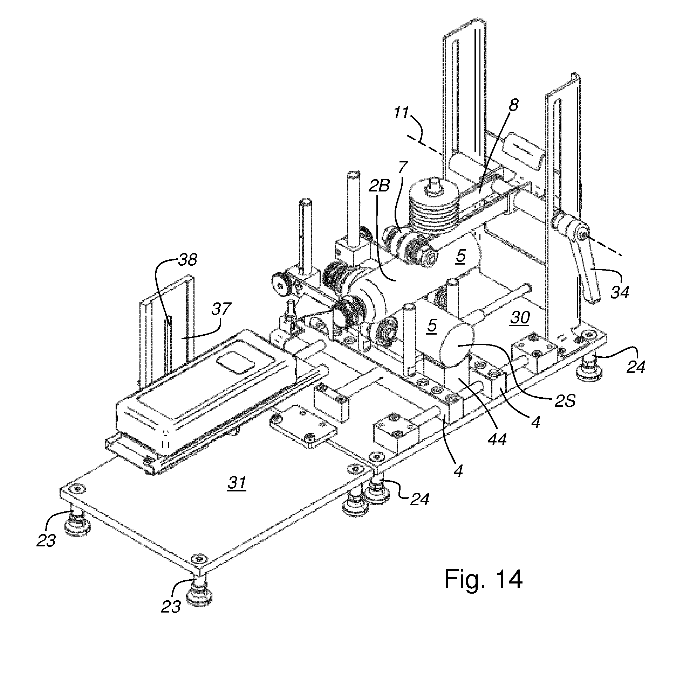

**Figur 14** zeigt eine perspektivische Ansicht der erfindungsgemäßen Vorrichtung 1, bei der Kunststoffflaschen in dem zweiten Modul 22 eingesetzt sind, damit die Haftreibung zwischen den beiden Kunststoffflaschen ermittelt werden kann. Der starre Kunststoffhohlkörper 2S ist zwischen den Klemmbacken 4 mit den dazu vorgesehenen Stäben 40 geklemmt. Die Tragarme 42 für den linear beweglichen Kunststoffhohlkörper 2B sind derart in der Höhe an den Stäben 41 verstellt, dass der linear bewegliche Kunststoffhohlkörper 2B auf den Lagern 7 aufliegt und dabei gleichzeitig die Mantelfläche 5 des starren Kunststoffhohlkörper 2S berührt. Mittels des Schnellspannhebels 34 ist die Schwenkachse 11 des Hebelarms 8 derart in der Höhe verstellt, dass der Schwenkarm 8 parallel zur Montageplatte des zweiten Moduls 22 liegt. Die Füße 23 des ersten Moduls 21 und die Füße 24 des zweiten Moduls 22 sind dabei derart in der Höhe H verstellt, dass die Montageplatte 31 des ersten Moduls 21 mit der Montageplatte 30 des zweiten Moduls 22 fluchtet, bzw. in einer Ebene zu liegen kommen.

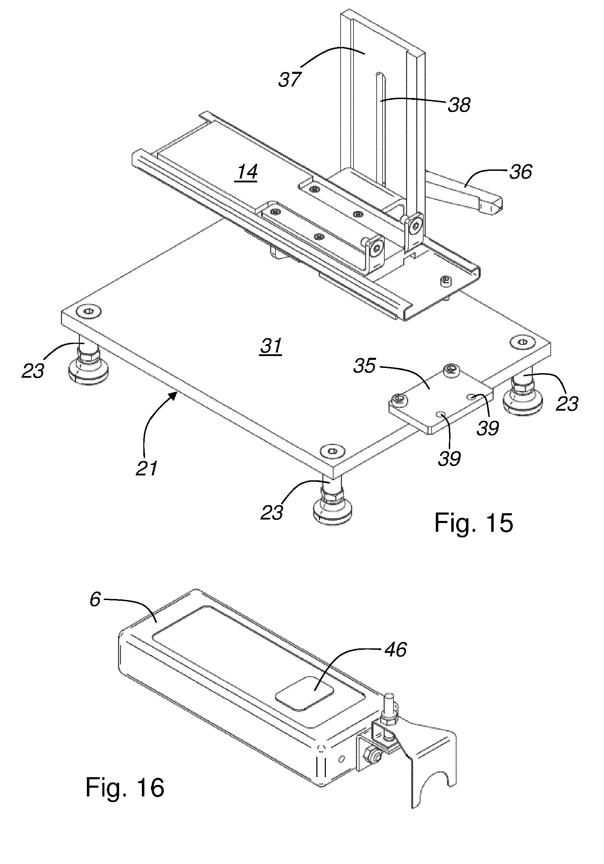

**Figur 15** zeigt eine perspektivische Ansicht des ersten Moduls 21 der erfindungsgemäßen Vorrichtung 1 zur Vermessung der Haftreibung zwischen Kunststoffflaschen. Die Montageplatte 31 des ersten Moduls 21 sitzt auf mehreren Füßen 23. An einer Halteplatte 37, die an der Montageplatte 31 angebracht ist, ist der Schlitten 14 in der Höhe verstellbar angebracht. Die Verstellung des Schlittens 14 erfolgt über ein in der Halteplatte 37 ausgeformtes Langloch 38. Die Fixierung in der vorbestimmten, bzw. gewünschten Höhe des Schlittens 14 erfolgt mit einem Schnellspannhebel 36. Entlang der Montageplatte 31 ist eine Lasche 35 angebracht, in der mindestens zwei Bohrungen 39 ausgebildet sind. Die Bohrungen wirken mit entsprechenden Stiften (nicht dargestellt) in der Montageplatte 31 des zweiten Moduls 22 zusammen. Somit ist es möglich, auf eine schnelle und einfache Weise eine Verbindung zwischen dem ersten Modul 21 und dem zweiten Modul 22 zu erreichen.

**Figur 16** zeigt eine perspektivische Ansicht der Kraftmesseinheit 6. Die Kraftmesseinheit 6 hat ein Display 46 ausgebildet, über das die maximale Kraft abgegeben werden kann, die notwendig ist, um die Haftreibung zwischen dem starren Kunststoffhohlkörper 2S und dem beweglichen Kunststoffhohlkörper 2B zu überwinden.

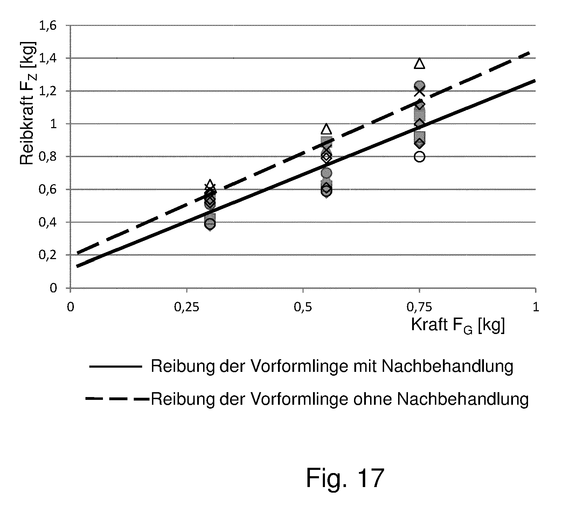

**Figur 17** zeigt eine graphische Darstellung der Abhängigkeit der Klebrigkeit zwischen Vorformlingen, die einer unterschiedlichen Nachbehandlung nach dem Spritz-Gießvorgang unterzogen worden sind. Auf der Abszisse ist die Kraft $F_G$ in Kilogramm aufgetragen, die auf den beweglichen Kunststoffhohlkörper 2B wirkt. Wie bereits in der vorangegangenen Beschreibung beschrieben, wird die Kraft $F_G$ auf den beweglichen K Kunststoffhohlkörper 2B mittels unterschiedlicher Gewichte auf den Schwenkarm 8 ausgerichtet. Auf der Ordinate ist die Zugkraft $F_Z$ aufgebracht, die nötig ist, um den beweglichen Kunststoffhohlkörper 2B vom starren Kunststoffhohlkörper 2S abzuziehen. Die durchgezogene Linie zeigt diejenigen Werte, die mit Vorformlingen gewonnen wurden, die nach dem Spritz-Gießvorgang einer Nachbehandlung unterzogen worden sind. Die gestrichelte Kurve zeigt diejenigen Werte für die Vorformlinge, die nach dem Spritz-Gießvorgang keiner Nachbehandlung unterzogen worden sind.

Für die maximale Zugkraft $F_{Z,manx}$ gilt:

$$F_{Z,\max} = \mu_H F_N + \mu_G F_N$$

Dabei steht $F_{Z,max}$ für die maximale Zugkraft, die der negativen maximalen Haltekraft $F_{R,max}$ entspricht. $\mu_H$ entspricht der Haftreibungszahl, die zwischen zwei Kunststoffhohlkörpern 2 einer gleichen Charge vorliegt und $\mu_G$ entspricht der Gleitreibungszahl.

Es zeigen sich, wie aus Figur 17 ersichtlich, deutliche Unterschiede. Die Vorformlinge, welche einer Nachbehandlung unterzogen worden sind, weisen eine niedrige Klebrigkeit untereinander auf. Mit der Kraftmesseinheit 6 wird eine maximale Zugkraft $F_{Z,max}$ gemessen, die nötig ist, um den beweglichen Kunststoffhohlkörper 2B gegenüber den starren Kunststoffhohlkörper 2S abzuziehen. Die maximale Kraft $F_{Z,max}$ ist von der Kraft $F_G$ abhängig, die über den schwenkbaren Hebelarm 8 auf den beweglichen Kunststoffhohlkörper 2B wirkt. Die Haftreibungszahl ermittelt sich, wenn man die ermittelten Punkte in einem Koordinatensystem überträgt und durch eine Gerade miteinander verbindet. Der Schnittpunkt der Geraden mit der Ordinate ist dann ein Maß für die Haftreibung beziehungsweise Klebrigkeit.

Mit der erfindungsgemäßen Vorrichtung 1 ist es möglich, Vorformlinge, bzw. Kunststoffflaschen mit jeder beliebigen Länge zu vermessen. Die erfindungsgemäße Vorrichtung hat dabei eine handliche Größe und kann ohne zerlegt zu werden, in einem Reisekoffer transportiert werden. Bei der Vorrichtung zur Vermessung der Haftreibung zwischen Kunststoffflaschen ist es von Vorteil, wenn das erste Modul 21 und das zweite Modul 22 voneinander getrennt werden können. Die erfindungsgemäßen Vorrichtungen sollen vorzugsweise auf der Baustelle und bei Kunden eingesetzt werden, wo es Probleme mit klebrigen Vorformlingen, bzw. klebrigen Kunststoffflaschen gibt. Ein weiteres Einsatzgebiet der erfindungsgemäßen Vorrichtung ist in der Wareneingangskontrolle für Vorformlinge, um somit die Rahmenbedingungen der Vorformlinge des Herstellers zu überprüfen und deren Qualität festzustellen. Anhand der überprüften Qualität der gelieferten Vorformlinge ist es dann möglich, Rückschlüsse auf die Verarbeitbarkeit der Vorformlinge in einer Streck-Blasmaschine, bzw. in einer gesamten Linie zu ziehen.

Die Erfindung wurde unter Bezugnahme auf bevorzugte Ausführungsformen beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

**Bezugszeichenliste:**

| | |
|---|---|
| 1 | Vorrichtung |
| 2 | Kunststoffhohlkörper |
| 2B | beweglicher Kunststoffhohlkörper |
| 2S | starr eingespannter Kunststoffhohlkörper |
| 3 | Halsbereich |
| 4 | Klemmbacken |
| 5 | zylindrischen Mantelfläche |
| 6 | Kraftmesseinheit |
| 7 | Lager |
| 8 | schwenkbarer Hebelarm |
| 8a | erster Arm |
| 8b | zweiter Arm |
| 9 | freies Ende |
| 10 | Gestell der Vorrichtung |
| 11 | Schwenkachse |
| 12 | Langloch |
| 13 | Gewichte |
| 13S | Gewichtstapel |
| 14 | Schlitten |
| 15 | starre Verbindung |
| 16 | zentrale Bohrung |

17 Dorn

18 Ventilverschluss

19 Mutter

20 starre Brücke

21 erstes Modul

22 zweites Modul

23 Fuß erstes Modul

24 Fuß zweites Modul

26 Formteil

27 Anschlagplatte

28 Stellschraube

29 Schnellspannhebel

30 Montageplatte zweites Modul

31 Montageplatte erstes Modul

32 Platte

33 Langloch

34 Schnellspannhebel

35 Lasche

36 Schnellspannhebel

37 Halteplatte

38 Langloch

39 Bohrungen

40 Stifte

41 Stab

42 Tragarm

43 Stellschraube

44 Auflageblock

46 Display

50 Tragring

B Basis der Vorrichtung

$F_G$ Kraft auf Kunststoffhohlkörper

$F_N$ Normalkraft

$F_R$ Haftreibungskraft

$F_Z$ Zugkraft

H Höhe

LS Längsachse

SW Schwenkrichtung

A-A Richtung der Bewegung der Klemmbacken

V-V vertikale Richtung

**Patentansprüche**

1. Vorrichtung (1) zum Bestimmen der Reibung zwischen Kunststoffhohlkörpern (2) aus gleicher Materialzusammensetzung, **dadurch gekennzeichnet, dass** mindestens ein Kunststoffhohlkörper (2S) parallel zu seiner Längsachse (LS) mittels beidseitiger Klemmbacken (4) starr eingespannt ist, dass auf den mindestens einen starr eingespannt Kunststoffhohlkörper (2S) ein Kunststoffhohlkörper (2B) aufliegt, der nicht parallel zur Längsachse (LS) des starr eingespannten Kunststoffhohlkörpers (2S) ist, dass der bewegliche Kunststoffhohlkörper (2B) einer Kraft ($F_G$) beaufschlagbar ist und an dem mindestens einen starr eingespannten Kunststoffhohlkörper (2S) mit dieser Kraft ($F_G$) aufliegt; und dass ein Halsbereich (3) des beweglichen Kunststoffhohlkörper (2B) mit einer Kraftmesseinheit (6) verbunden ist.

2. Vorrichtung (1) nach Anspruch 1, wobei die Kunststoffhohlkörpern (2) Vorformlinge sind und zwei Vorformlinge zwischen den beidseitigen Klemmbacken (4) starr eingespannt sind und wobei ein Vorformling senkrecht zur Längsachse der beiden starr eingespannten Vorformlinge beweglich ist und auf den beiden starr eingespannten Vorformlingen aufliegt.

3. Vorrichtung (1) nach Anspruch 1, wobei die Kunststoffhohlkörper (2) aus Vorformlingen hergestellte Kunststoffflaschen sind und eine Kunststoffflasche zwischen den beidseitigen Klemmbacken (4) starr eingespannt ist und wobei eine Kunststoffflasche senkrecht zur Längsachse der starr eingespannten Kunststoffflasche beweglich ist und auf der starr eingespannten Kunststoffflasche aufliegt.

4. Vorrichtung (1) nach Anspruch 3, wobei die Kunststofflasche mit einem Innendruck (P) beaufschlagt ist.

5. Vorrichtung (1) nach einem der vorangehenden Ansprüche, wobei ein schwenkbarer Hebelarm (8) vorgesehen ist, der an einem freien Ende (9F) mindestens ein frei drehbares Lager (7) trägt und über eine Schwenkachse (11) mit einem Gestell (10) der Vorrichtung (1) schwenkbar und über ein Langloch (12) in einer Höhe (H) bezüglich einer Basis (1B) der Vorrichtung (1) linear verstellbar verbunden ist, dass das mindestens eine frei drehbares Lager (7) am beweglichen Kunststoffhohlkörper (2B) anliegt und, wobei auf dem schwenkbaren Hebelarm (8) mehrere Gewichte (13) aufsetzbar sind.

6. Vorrichtung (1) nach einem der vorangehenden Ansprüche, wobei ein Schlitten (14) mit der Vorrichtung (1) verbindbar ist, wobei der Schlitten (14) in einer vertikalen Richtung (V) verstellbar ist und wobei die Kraftmesseinheit (6) am Schlitten linear in Richtung einer Zugkraft ($F_Z$) verschiebbar ist.

7. Vorrichtung (1) nach einem der vorangehenden Ansprüche, wobei die Kraftmesseinheit (6) und der Halsbereich (3) des beweglichen Kunststoffhohlkörpers (2B) mit einer starren Verbindung koppelbar sind.

8. Vorrichtung (1) nach einem der vorangehenden Ansprüche, wobei die Vorrichtung (1) aus einem ersten Modul 21 und einem zweiten Modul (22) besteht, die miteinander lösbar verbunden sind.

9. Vorrichtung (1) nach Anspruch 8, wobei das erste Modul (21) die Kraftmesseinheit (6) trägt.

10. Vorrichtung (1) nach Anspruch 8, wobei das zweite Modul (22) die zu vermessenden Kunststoffhohlkörper (2), die Klemmbacken (4) für die starr eingespannten Kunststoffhohlkörper (2S) und den schwenkbaren Hebelarm (8) zur Beaufschlagung des beweglichen Kunststoffhohlkörpers (2B) mit einer Kraft ($F_G$) trägt.

11. Verfahren zum Bestimmen der Reibung zwischen Kunststoffhohlkörpern (2) aus gleicher Materialzusammensetzung, **gekennzeichnet durch** die folgenden Schritte:

- dass mindestens ein Kunststoffhohlkörper (2S) starr eingespannt wird;
- dass ein beweglicher Kunststoffhohlkörper (2B) derart auf den mindestens einen starr eingespannten Kunststoffhohlkörper (2S) gesetzt wird, dass deren Längsachsen (LS) nicht parallel zueinander stehen;
- dass ein Halsbereich (3) des beweglichen Kunststoffhohlkörpers (2B) mit einer Kraftmesseinheit (6) starr verbunden wird;
- dass der bewegliche Kunststoffhohlkörper (2B) mit einer Kraft ($F_G$) beaufschlagt wird,
- dass die Kraftmesseinheit (6) linear verschoben wird und ein Maximum einer Zugkraft ($F_Z$) ermittelt wird, die derjenigen Kraft entspricht, bei der der bewegliche Kunststoffhohlkörper (2B) an mindestens einem starr eingespannten Kunststoffhohlkörper (2S) zu gleiten beginnt; und
- dass das Maximum einer Zugkraft ($F_Z$) bei unterschiedlichen Kräften ($F_G$) bestimmt wird, mit denen der bewegliche Kunststoffhohlkörper (2B) beaufschlagt wird.

12. Verfahren nach Anspruch 11, wobei die Maxima der Zugkraft ($F_Z$) als Funktion der Kraft ($F_G$) aufgetragen wird, mit der der bewegliche Kunststoffhohlkörper (2B) beaufschlagt wird, die Punkte im Koordinatensystem miteinander durch eine Gerade verbunden werden und der resultierende Schnittpunkt der Geraden mit der Ordinate als Maß für die Haftreibung beziehungsweise Klebrigkeit abgelesen werden kann.

13. Verfahren nach den Ansprüchen 11 - 12, wobei die Kunststoffhohlkörper (2) Vorformlinge sind und zwei Vorformlinge starr eingespannt werden und wobei ein Vorformling senkrecht zur Längsachse der beiden starr eingespannten Vorformlinge zur Bestimmung der maximalen Zugkraft ($F_Z$) bewegt wird.

14. Verfahren nach den Ansprüchen 11 - 12 wobei die Kunststoffhohlkörper (2) aus Vorformlingen hergestellte Kunststoffflaschen sind und eine Kunststoffflasche starr eingespannt wird und wobei eine Kunststoffflasche senkrecht zur Längsachse der starr eingespannten Kunststoffflasche zur Bestimmung der maximalen Zugkraft ($F_Z$) bewegt wird.

15. Verfahren nach Anspruch 14, wobei die Kunststofflaschen mit einem Innendruck (P) beaufschlagt und am Halsbereich (3) mit einem Ventilverschluss (18) verschlossen werden.

2
50
$F_G$
5
2B
LS
$F_Z$
$F_R$
2
2
2S
LS
$F_N$
LS
2S

Fig. 1

2
2B
$F_G$
5
LS
18
$F_R$
$F_Z$
3
2S
LS
2
$F_N$

Fig. 2

1
$F_G$
13
9
7
8 SW
11
6
50
2
2B
3
$F_Z$
15
V-V
14
H
4
4
10
B
2S
2S
A-A
A-A

Fig. 3

1
$F_G$
13
8
SW
12
9
7
2
2B
6
3
15
$F_Z$
11
V-V
14
2
H
4
4
10
H
B
A-A
2S
A-A

Fig. 4

11
8
26
13
1
7
28
2B
11
6
22
4
24
2S
4
21
24
23

Fig. 5

11
26
28
4
4
24
30
24
22
29
27
24

Fig. 6

16
13S
13
17
19
13
8
11
9
7

Fig. 7

Fig. 8

17
8a
11
7
8
8b
20
9

Fig. 9

31
21
33
32
14
6
15
31
14
21
6
33
32
15

Fig. 10

Fig. 11

12
26
41
40
8
41
1
42
11
42
34
7
37
38
6
7
30
24
14
40
4
35
4
40
22
24
31
23
23
21
23

Fig. 12

36
43
44
43
22
28
35
42
42
6
30
31
21
7
4
44
4
7

Fig. 13

11
8
7
2B
5
38
37
5
30
34
24
2S
4
44
4
31
24
23
23

Fig. 14

37
38
14
36
31
35
23
23
39
39
21
23

Fig. 15

6
46

Fig. 16

1,6
1,4
1,2
1
0,8
0,6
0,4
0,2
0
Reibkraft F_Z [kg]
0
0,25
0,5
0,75
1
Kraft F_G [kg]
Reibung der Vorformlinge mit Nachbehandlung
Reibung der Vorformlinge ohne Nachbehandlung

Fig. 17

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2007094704 A1
- GB 2187560 A
- US 20020194895 A1
- DD 234933 A1